(19) Europäisches Patentamt

European Patent Office

Office européen des brevets

(11) **EP 4 487 890 A1**

## (12) EUROPEAN PATENT APPLICATION

(43) Date of publication:
08.01.2025 Bulletin 2025/02

(21) Application number: 23183866.5

(22) Date of filing: 06.07.2023

(51) International Patent Classification (IPC):
**A61M 5/32** (2006.01)

(52) Cooperative Patent Classification (CPC):
**A61M 5/32; A61M 5/329**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Becton Dickinson France
38800 Le Pont-de-Claix (FR)**
• **Becton, Dickinson and Company
Franklin Lakes, NJ 07417-1880 (US)**

(72) Inventors:
• **LEANG, Sandra
38170 Seyssinet Pariset (FR)**
• **DUINAT, Brigitte
38100 Grenoble (FR)**
• **FREMON, Benoit
38410 Saint Martin d'Uriage (FR)**
• **PRAIS, Alfred, W.
New Jersey 07421 (US)**

(74) Representative: **Germain Maureau
12, rue Boileau
69006 Lyon (FR)**

## (54) THIN WALL CANNULA

(57) A medical injection cannula configured for use with a syringe includes a sidewall defining a lumen and having a proximal end and a distal end, and a beveled tip defining an opening to the lumen. A length-to-diameter ratio of the cannula is defined by a ratio

$$\frac{L_{hydrodynamic\ theoretical\ needle}}{D_{needle\_inner}^{4}}$$

, wherein $L_{hydrodynamic\ theoretical\ needle}$ is a theoretical hydrodynamic length of the cannula, and $D_{needle\_inner}$ is an inner diameter of the sidewall of the cannula. The length-to-diameter ratio is between $8.5 \times 10^{11}$ and $8.5 \times 10^{12}$ m$^{-3}$.

FIG.1

EP 4 487 890 A1

# EP 4 487 890 A1

**Description**

BACKGROUND OF THE INVENTION

**Field of the Invention**

The present disclosure relates to cannulas for syringes and other medical devices.

**Description of Related Art**

Cannulas are a ubiquitous component of modern medical care. Cannulas are used for example, to pierce a patient's skin to create a vascular access site for the administration of medicaments and other fluids (e.g. saline), or to withdraw blood from the patient. To reduce patient discomfort and the risk of injury, the outer diameter of cannulas is desirably minimized. However, the lumen of the cannula must be of sufficient diameter to allow the medicament or other fluid to flow through the cannula at an adequate rate, whatever the viscosity of the injected drug. As such, it is desirable to produce cannula with thin sidewalls to minimize the outer diameter while maximizing the inner diameter.

SUMMARY

Embodiments of the present disclosure are directed to a medical injection cannula configured for use with a syringe. The cannula includes a sidewall defining a lumen and having a proximal end and a distal end, and a beveled tip defining an opening to the lumen, wherein a length-to-diameter ratio of the cannula is defined by a ratio $\dfrac{L_{hydrodynamic\ theoretical\ needle}}{D_{needle\_inner}{}^4}$, wherein $L_{hydrodynamic\ theoretical\ needle}$ is a theoretical hydrodynamic length of the cannula, and $D_{needle\_inner}$ is an inner diameter of the sidewall of the cannula, and wherein the length-to-diameter ratio is comprised between $8.5 \times 10^{11}$ and $8.5 \times 10^{12}$ $m^{-3}$.

In accordance with an embodiment, the hydrodynamic length of the cannula $L_{hydrodynamic}$ extends from a break of a transition region of the syringe to a proximal end of the opening of the beveled tip.

In accordance with an embodiment, the outer diameter of the sidewall corresponds to a cannula standard for a 27G, 29G or 30G cannula.

In accordance with an embodiment, the outer diameter of the sidewall is for the 27G cannula and the ratio $\dfrac{L_{hydrodynamic\ theoretical\ needle}}{D_{needle\_inner}{}^4}$, is comprised between $8.5 \times 10^{11}$ $m^{-3}$ and $1.5 \times 10^{12}$ $m^{-3}$.

In accordance with an embodiment, the outer diameter of the sidewall is for the 29G cannula and the ratio $\dfrac{L_{hydrodynamic\ theoretical\ needle}}{D_{needle\_inner}{}^4}$, is comprised between $2 \times 10^{12}$ $m^{-3}$ and $4.5 \times 10^{12}$ $m^{-3}$.

In accordance with an embodiment, the outer diameter of the sidewall is for the 30G cannula and the ratio $\dfrac{L_{hydrodynamic\ theoretical\ needle}}{D_{needle\_inner}{}^4}$, is comprised between $4.5 \times 10^{12}$ $m^{-3}$ and $8.5 \times 10^{12}$ $m^{-3}$.

In accordance with an embodiment, the outer diameter of the sidewall is substantially constant between the proximal end and the distal end.

Also provided herein is a medical injection assembly including a syringe having a sidewall and a distal end, and a cannula connected to the distal end. The cannula further includes a sidewall defining a lumen and having a proximal end and a distal end and a beveled tip defining an opening to the lumen, wherein a flow factor $K_1$ of a fluid through the lumen of the cannula is approximated by the equation $\dfrac{8\,\pi\,D_{barrel}{}^4\,L_{hydrodynamic\ theoretical\ needle}}{D_{needle\_inner}{}^4}$, wherein $D_{barrel}$ is an inner diameter of a sidewall of the syringe, $L_{hydrodynamic}$ is a hydrodynamic length of the cannula, and $D_{hydrodynamic}$ is an empirically derived inner diameter of the sidewall of the cannula, and wherein $K_1$ is comprised between 34,000 and $1.3 \times 10^6$ meters.

In accordance with an embodiment, the inside volume of the syringe is comprised between 1 mL and 2.25 mL.

In accordance with an embodiment, the sidewall has a transition region wherein the inner diameter transitions to a break at which an inner diameter of the sidewall is approximately equal to the inner diameter of the cannula.

In accordance with an embodiment, the hydrodynamic length of the cannula extends from the break of the syringe to a proximal end of the opening of the beveled tip.

2

In accordance with an embodiment, the outer diameter of the sidewall corresponds to a cannula standard for a 27G, 29G or 30G cannula.

In accordance with an embodiment, the outer diameter of the sidewall of the cannula is substantially constant between the proximal end and the distal end.

In accordance with an embodiment, the syringe further includes a stopper slidably disposed within the sidewall of the syringe.

Further details and advantages of the various examples described in detail herein will become clear upon reviewing the following detailed description of the various examples in conjunction with the accompanying drawing figures.

## BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:

FIG. 1 is a schematic of a cannula attached to a distal end of a syringe barrel.
FIG. 2 is a top view of the cannula of FIG. 1.
FIG. 3 is a graph illustrating acceptable length-to-diameter ratios of 27G, 29G and 30G cannulas of 8mm exposed length, according to aspects of the disclosure.
FIG. 4 is a graph illustrating acceptable flow factors $K_1$ of 1mLL and 2.25mLL syringes having a 27G, 29G or 30G cannula of 8mm exposed length, according to aspects of the disclosure.

Corresponding reference characters indicate corresponding parts throughout the several views. The exemplifications set out herein illustrate exemplary embodiments of the disclosure, and such exemplifications are not to be construed as limiting the scope of the disclosure in any manner.

## DETAILED DESCRIPTION

The following description is provided to enable those skilled in the art to make and use the described aspects contemplated for carrying out the disclosure. Various modifications, equivalents, variations, and alternatives, however, will remain readily apparent to those skilled in the art. Any and all such modifications, variations, equivalents, and alternatives are intended to fall within the spirit and scope of the present disclosure.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary aspects of the invention. Hence, specific dimensions and other physical characteristics related to the aspects disclosed herein are not to be considered as limiting.

All numbers used in the specification and claims are to be understood as being modified in all instances by the term "about". The terms "approximately", "about", and "substantially" mean a range of plus or minus ten percent of the stated value. Further, the term "substantially equal" and like terms mean that the compared values or dimensions are within a range of plus or minus ten percent of one another.

The terms "first", "second", and the like are not intended to refer to any particular order or chronology, but refer to different conditions, properties, or elements.

As used herein with reference to an injection apparatus such as a syringe, the term "proximal" refers to an end of the apparatus farthest from the outlet, or to a direction toward the end of the apparatus farthest from the outlet. As used herein with reference to an injection apparatus such as a syringe, the term "distal" refers to an end of the device or apparatus closest to the outlet, or to a direction toward the end of the apparatus closest to the outlet.

As used herein, "at least one of" is synonymous with "one or more of". For example, the phrase "at least one of A, B, and C" means any one of A, B, or C, or any combination of any two or more of A, B, or C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C.

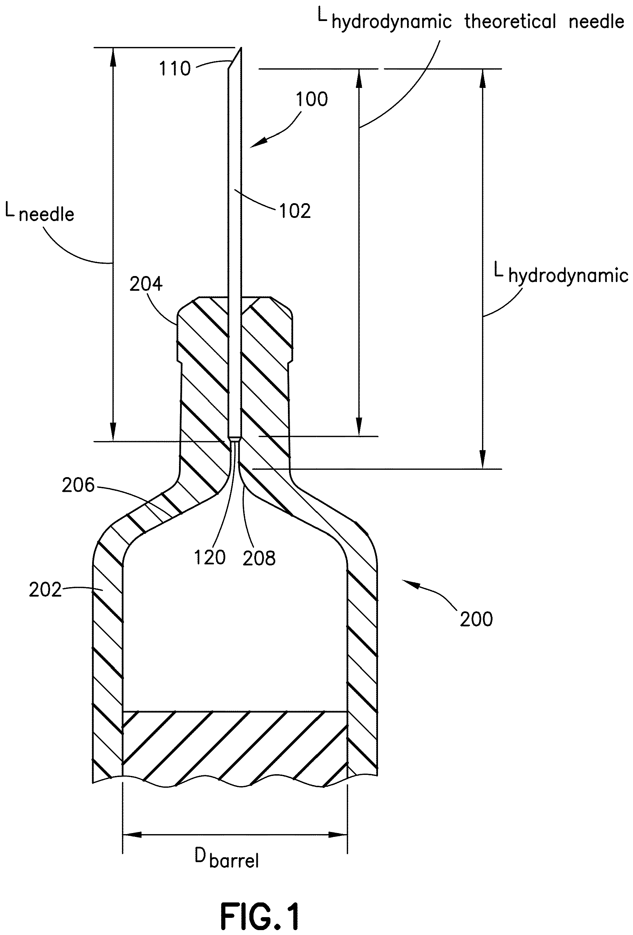

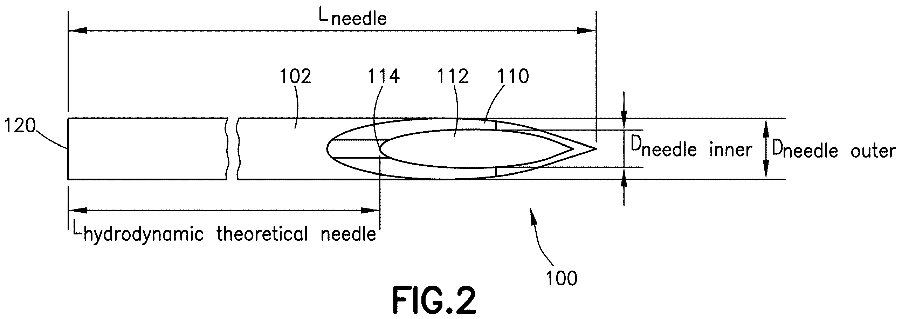

Referring now to FIGS. 1 and 2, a cannula 100 in accordance with the present disclosure includes a sidewall 102 extending between a distal end 110 and a proximal end 120. The sidewall 102 has an outer diameter $D_{needle\_outer}$ and an inner diameter $D_{needle\_inner}$, with a difference between the outer diameter $D_{needle\_outer}$ and the inner diameter $D_{needle\_inner}$ defining a wall thickness. The inner diameter $D_{needle\_inner}$ of the sidewall 102 defines a lumen whose cross-section is substantially circular and through which a medicament, blood, or other fluid can flow. The outer diameter

$D_{needle\_outer}$ of the sidewall 102 may be equivalent to industry-standard nominal sizes for a selected cannula gauge (G), with the gauge size defining the limits for the outer diameter. As non-limiting examples, a 27G cannula will have an outer diameter of between 0.400 and 0.420 mm, a 29G cannula will have an outer diameter of between 0.324 and 0.351 mm, and a 30G cannula will have an outer diameter of between 0.298 and 0.320 mm. The outer diameter $D_{needle\_outer}$ of the sidewall 102 may be substantially constant between the proximal end 120 and the distal end 110. The distal end 110 may include a beveled tip configured for piercing a patient's skin to create a vascular access site. The beveled tip defines an opening 112 into the lumen. The opening has a proximal end 114. The proximal end 120 of the cannula 100 is configured for connection to a distal end 204 of a syringe 200. The overall length $L_{needle}$ of the cannula 100 extends from the proximal-most end of the sidewall 102 to the distal-most end of the sidewall 102, including the length of the beveled tip of the distal end 110.

As shown in FIG. 1, the syringe 200 (which may be formed of plastic or glass, typically glass) includes a sidewall 202 having an inner diameter $D_{barrel}$. The inner diameter $D_{barrel}$ depends on the volume of the barrel and the associated volume of drug to be injected. For instance, the barrel may be a 1mLL (1 mm long) or 2.25mLL (2.25 mm long) barrel, with the 1mLL barrel having an inner diameter $D_{barrel}$ of 6.35mm +/- 0.01mm and the 2.25mLL barrel having an inner diameter $D_{barrel}$ of 8.65mm +/- 0.2mm. The sidewall 202 tapers toward the distal end 204 of the syringe 200 where the cannula 100 is connected (e.g., glued). In particular, the sidewall 202 tapers in a transition region 206 from the inner diameter $D_{barrel}$ to a smaller diameter slightly lower than the outer diameter $D_{needle\_outer}$ of the cannula 100. The transition region 206 ends at a break 208, at which point the inner diameter of the sidewall 202 is approximately equal to the inner diameter $D_{needle\_inner}$ of the cannula 100. An elastomeric stopper 220 is slidably disposed within the sidewall 202 and can be advanced distally to expel fluid from the syringe 200 through the cannula 100. The stopper 220 may be affixed to the end of a plunger (not shown) that can be depressed by a clinician's finger(s).

Three different lengths of the cannula 100 are significant to measuring and characterizing the flow rate of a fluid through the lumen of the cannula 100. First, the aforementioned overall length $L_{needle}$ extends from the proximal-most end of the sidewall 102 to the distal-most end of the sidewall 102. Second, the theoretical hydrodynamic length $L_{hydrodynamic\ theoretical\ needle}$ extends from the proximal-most end of the sidewall 102 to the proximal end 114 of the opening 112 of the beveled tip. Third, the actual hydrodynamic length $L_{hydrodynamic}$ extends from the break 208 of the syringe 200 (where the diameter of the barrel is the smallest) to the proximal end 114 of the opening 112 of the beveled tip.

Injection force required to inject a fluid from the syringe 200 through the cannula 100 is a complex calculation involving many factors. However, this calculation can be simplified for many relevant applications so long as stopper speed within the syringe is not abnormally high, and so long as dynamic viscosity of the fluid injected in not abnormally low. In particular, the calculation of an injection force F can be simplified as shown below in Equation 1:

$$\text{Equation 1: } F = \frac{8\,\pi\,D_{barrel}{}^4\,L_{hydrodynamic}}{D_{hydrodynamic}{}^4} \times \eta \times v_{stopper} + R,$$

where F is injection force, $\eta$ is viscosity of the fluid injected, $v_{stopper}$ is the speed of the stopper in the syringe 200, and $R$ is force due to friction of the stopper.

The terms in Equation 1 that are derived from the physical dimensions of the cannula 100 and barrel 200 can be gathered into a flow factor $K_1$, as shown below in Equation 2:

$$\text{Equation 2: } K_1 = \frac{8\,\pi\,D_{barrel}{}^4\,L_{hydrodynamic}}{D_{hydrodynamic}{}^4}.$$

In other embodiments, where a theoretical hydrodynamic needle length $L_{hydrodynamic\ theoretical\ needle}$ and inner diameter $D_{needle\_inner}$ define the dimension of the cannula 100, the flow factor $K_1$, may be as shown below in Equation 3:

$$\text{Equation 3: } K_1 = \frac{8\,\pi\,D_{barrel}{}^4\,L_{hydrodynamic\ theoretical\ needle}}{D_{needle\_inner}{}^4}.$$

The flow factor $K_1$ is presented in units of distance and, for most cannulas used in medical devices, is typically in a range of 10,000 meters and 10,000,000 meters. The flow factor $K_1$ is related to injection time, with a relatively lower flow factor $K_1$ correlating to a faster injection time and a relatively higher flow factor $K_1$ correlating to a longer injection time for the same volume of fluid injected. In order to optimize the injection of a drug, it is desirable to have a lower factor $K_1$. Indeed, when the factor $K_1$ is lowered, the injection force required to inject the drug contained into the syringe is lowered too. In lowering the required injection force, the injection is easier to perform and/or a more viscous drug may be inject into a patient's skin without considerably increasing the required injection force.

In addition to the flow factor $K_1$, the cannula 100 according to the present disclosure can be characterized by what may generally be termed a "length-to-diameter ratio" of the cannula - with the length-to-diameter ratio defined herein as a ratio of the theoretical hydrodynamic needle length to the fourth power of the inner diameter ($L_{hydrodynamic\ theoretical\ needle} / D_{needle\_inner}^4$) or as a ratio of the actual hydrodynamic needle length to the fourth power of the hydrodynamic diameter ($L_{hydrodynamic} / D_{hydrodynamic}^4$).

The inner diameter $D_{needle\_inner}$ of the cannula 100 is typically ascertained from a dimensional drawing of the cannula 100, and, due to manufacturing limitations, is not necessarily reflective of a measurement from the actual cannula 100. In order to measure small diameter bores such as cannula lumens, gauge pins are typically used. However, gauge pins have their own limitations, notably in that gauge pins are incapable of reliably measuring a tapered bore and other inconsistencies in the bore introduced by manufacturing. Thus, instead of relying on physically measuring the inner diameter $D_{needle\_inner}$ of the cannula 100, the hydrodynamic diameter $D_{hydrodynamic}$ can be experimentally derived by pushing fluid through the cannula at a known or deduced flow rate. In some embodiments, the flow rate may be deduced by measuring the mass of fluid during a given time period and by knowing the density of the fluid. Based on this flow rate, the ratio of the actual hydrodynamic needle length to the fourth power of the hydrodynamic diameter ($L_{hydrodynamic} / D_{hydrodynamic}^4$) can be deduced via the fluid viscosity and test pressure. If the actual hydrodynamic length $L_{hydrodynamic}$ of the needle is known, the hydrodynamic diameter $D_{hydrodynamic}$ may then be deduced.

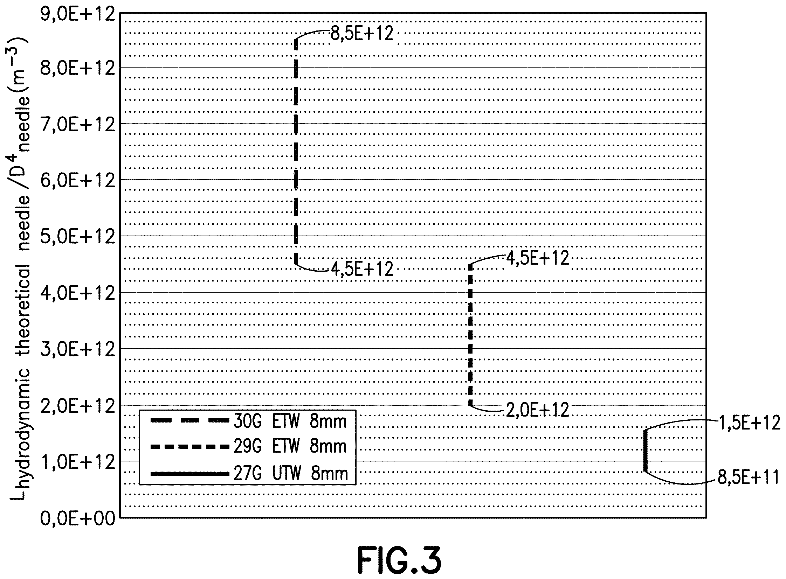

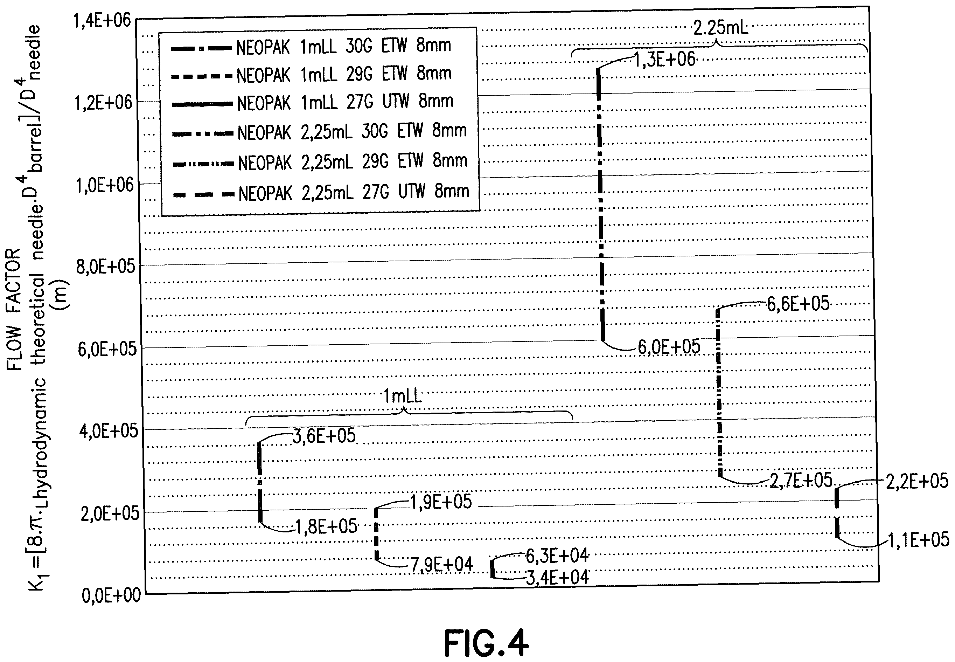

Referring now to FIGS. 3 and 4, graphs are providing illustrating the length-to-diameter ratio (i.e., the ratio of the theoretical hydrodynamic needle length to the fourth power of the inner diameter, $L_{hydrodynamic\ theoretical\ needle} / D_{needle\_inner}^4$) and the flow factor $K_1$, respectively, for various syringes that include a cannula having an 8mm exposed length, according to aspects of the disclosure.

Referring first to FIG. 3, an acceptable range of the length-to-diameter ratio ($L_{hydrodynamic\ theoretical\ needle} / D_{needle\_inner}^4$) is provided for each of a 27G, 29G and 30G cannula. As shown therein, the length-to-diameter ratio of the 27G cannula is between $8.5 \times 10^{11}$ m$^{-3}$ and $1.5 \times 10^{12}$ m$^{-3}$, the length-to-diameter ratio of the 29G cannula is between $2 \times 10^{12}$ m$^{-3}$ and $4.5 \times 10^{12}$ m$^{-3}$, and the length-to-diameter ratio of the 30G cannula is between $4.5 \times 10^{12}$ m$^{-3}$ and $8.5 \times 10^{12}$m$^{-3}$. An acceptable range of the length-to-diameter ratio across all of the or 27G, 29G and 30G cannulas is thus between $8.5 \times 10^{11}$ and $8.5 \times 10^{12}$ m$^{-3}$, according to aspects of the present disclosure.

Referring next to FIG. 4, an acceptable range of a flow factor $K_1$ is provided for 1mLL syringes having a 27G, 29G or 30G cannula and for 2.25 mLL syringes having a 27G, 29G or 30G cannula. As shown therein, the flow factor $K_1$ of the 1mLL syringes is between 34,000m and 63,000m for the 27G cannula, 79,000m and $1.9 \times 10^5$m for the 29G cannula, and $1.8 \times 10^5$m and $3.6 \times 10^5$m for the 30G cannula, while the flow factor $K_1$ of the 2.25mLL syringes is between $1.1 \times 10^5$m and $2.2 \times 10^5$m for the 27G cannula, $2.7 \times 10^5$m and $6.6 \times 10^5$m for the 29G cannula, $6.0 \times 10^5$m and $1.3 \times 10^6$m for the 30G cannula. An acceptable range of the flow factor $K_1$ across the 1mLL and 2.25mLL syringes and all of the 27G, 29G and 30G cannulas is thus between 34,000 and $1.3 \times 10^6$ meters.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

**Claims**

1. A medical injection cannula configured for use with a syringe, the cannula comprising:

    a sidewall defining a lumen and having a proximal end and a distal end; and
    a beveled tip defining an opening to the lumen,
    wherein a length-to-diameter ratio of the cannula is defined by a ratio:

$$\frac{L_{hydrodynamic\ theoretical\ needle}}{D_{needle\_inner}^4}$$

    wherein $L_{hydrodynamic\ theoretical\ needle}$ is a theoretical hydrodynamic length of the cannula, and $D_{needle\_inner}$ is an inner diameter of the sidewall of the cannula; and
    wherein the length-to-diameter ratio is comprised between $8.5 \times 10^{11}$ and $8.5 \times 10^{12}$ m$^{-3}$.

2. The medical injection cannula of claim 1, wherein the hydrodynamic length of the cannula $L_{hydrodynamic}$ extends from a break of a transition region of the syringe to a proximal end of the opening of the beveled tip.

3. The medical injection cannula of claim 1 or 2, wherein the outer diameter of the sidewall corresponds to a cannula standard for a 27G, 29G or 30G cannula.

4. The medical injection cannula of claim 1 to 3, wherein the outer diameter of the sidewall is for the 27G cannula and the ratio $\dfrac{L_{hydrodynamic\ theoretical\ needle}}{D_{needle\_inner}^{4}}$, is comprised between $8.5 \times 10^{11}$ m$^{-3}$ and $1.5 \times 10^{12}$ m$^{-3}$.

5. The medical injection cannula of claim 1 to 3, wherein the outer diameter of the sidewall is for the 29G cannula and the ratio $\dfrac{L_{hydrodynamic\ theoretical\ needle}}{D_{needle\_inner}^{4}}$, is comprised between $2 \times 10^{12}$ m$^{-3}$ and $4.5 \times 10^{12}$ m$^{-3}$.

6. The medical injection cannula of claim 1 to 3, wherein the outer diameter of the sidewall is for the 30G cannula and the ratio $\dfrac{L_{hydrodynamic\ theoretical\ needle}}{D_{needle\_inner}^{4}}$, is comprised between $4.5 \times 10^{12}$ m$^{-3}$ and $8.5 \times 10^{12}$ m$^{-3}$.

7. The medical injection cannula of any of claims 1 to 6, wherein the outer diameter of the sidewall is substantially constant between the proximal end and the distal end.

8. A medical injection assembly, comprising:

   a syringe having a sidewall and a distal end; and
   a cannula connected to the distal end, the cannula comprising:

   a sidewall defining a lumen and having a proximal end and a distal end; and
   a beveled tip defining an opening to the lumen,
   wherein a flow factor $K_1$ of a fluid through the lumen of the cannula is approximated by the equation:

$$\frac{8\,\pi\,D_{barrel}^{\,4}\,L_{hydrodynamic\ theoretical\ needle}}{D_{needle\_inner}^{\,4}}$$

   wherein $D_{barrel}$ is an inner diameter of a sidewall of the syringe, $L_{hydrodynamic}$ is a hydrodynamic length of the cannula, and $D_{hydrodynamic}$ is an empirically derived inner diameter of the sidewall of the cannula; and
   wherein $K_1$ is comprised between 34,000 and $1.3 \times 10^6$ meters.

9. The assembly of claim 8 wherein the inside volume of the syringe is comprised between 1 mL and 2.25 mL.

10. The assembly of claim 8 or 9, wherein the sidewall has a transition region wherein the inner diameter transitions to a break at which an inner diameter of the sidewall is approximately equal to the inner diameter of the cannula.

11. The assembly of any of claims 8 to 10, wherein the hydrodynamic length of the cannula extends from the break of the syringe to a proximal end of the opening of the beveled tip.

12. The assembly of any of claims 8 to 11, wherein the outer diameter of the sidewall corresponds to a cannula standard for a 27G, 29G or 30G cannula.

13. The assembly of any of claims 8 to 12, wherein the outer diameter of the sidewall of the cannula is substantially constant between the proximal end and the distal end.

14. The assembly of any of claims 8 to 13, further including a stopper slidably disposed within the sidewall of the syringe.

FIG.1

FIG.2

FIG.3

FIG.4

Europäisches
Patentamt

European
Patent Office

Office européen
des brevets

# EUROPEAN SEARCH REPORT

Application Number

EP 23 18 3866

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/268987 A1 (YU MINHONG [US]) 27 August 2020 (2020-08-27) * figures 1-9 * * paragraph [0041] - paragraph [0047] * ----- | 1-14 | INV. A61M5/32 |
| X | US 2017/216529 A1 (DONOVAN MARTIN [US]) 3 August 2017 (2017-08-03) * figures 1-2 * * paragraph [0071] - paragraph [0091] * ----- | 1-14 | |
| X | US 2002/138050 A1 (SMEDEGAARD JORGEN K [DK] ET AL) 26 September 2002 (2002-09-26) * the whole document * ----- | 1-8, 10-13 | |

TECHNICAL FIELDS
SEARCHED (IPC)

A61M

The present search report has been drawn up for all claims

| Place of search | Date of completion of the search | Examiner |
|---|---|---|
| Munich | 12 December 2023 | Delmotte, Pierre |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

........................................................................

& : member of the same patent family, corresponding
  document

EPO FORM 1503 03.82 (P04C01)

2

## ANNEX TO THE EUROPEAN SEARCH REPORT
## ON EUROPEAN PATENT APPLICATION NO.

**EP 23 18 3866**

This annex lists the patent family members relating to the patent documents cited in the above-mentioned European search report.
The members are as contained in the European Patent Office EDP file on
The European Patent Office is in no way liable for these particulars which are merely given for the purpose of information.

**12-12-2023**

| Patent document cited in search report | | | Publication date | Patent family member(s) | | | Publication date |
|---|---|---|---|---|---|---|---|
| US 2020268987 | A1 | | 27-08-2020 | US | 2020268987 | A1 | 27-08-2020 |
| | | | | WO | 2020176391 | A1 | 03-09-2020 |
| US 2017216529 | A1 | | 03-08-2017 | AU | 2015300944 | A1 | 02-03-2017 |
| | | | | CA | 2957399 | A1 | 11-02-2016 |
| | | | | CN | 106573106 | A | 19-04-2017 |
| | | | | EP | 3185932 | A1 | 05-07-2017 |
| | | | | EP | 3871709 | A1 | 01-09-2021 |
| | | | | JP | 6982495 | B2 | 17-12-2021 |
| | | | | JP | 2017522983 | A | 17-08-2017 |
| | | | | JP | 2021010767 | A | 04-02-2021 |
| | | | | KR | 20170040330 | A | 12-04-2017 |
| | | | | US | 2017216529 | A1 | 03-08-2017 |
| | | | | WO | 2016022831 | A1 | 11-02-2016 |
| US 2002138050 | A1 | | 26-09-2002 | AT | E179622 | T1 | 15-05-1999 |
| | | | | AU | 708225 | B2 | 29-07-1999 |
| | | | | BR | 9612260 | A | 13-07-1999 |
| | | | | CA | 2241026 | A1 | 03-07-1997 |
| | | | | CN | 1205645 | A | 20-01-1999 |
| | | | | CZ | 289840 | B6 | 17-04-2002 |
| | | | | DE | 29624204 | U1 | 08-02-2001 |
| | | | | DE | 69602379 | T2 | 30-12-1999 |
| | | | | DK | 0861102 | T3 | 01-11-1999 |
| | | | | EP | 0861102 | A1 | 02-09-1998 |
| | | | | ES | 2134021 | T3 | 16-09-1999 |
| | | | | GR | 3030763 | T3 | 30-11-1999 |
| | | | | HU | 9901087 | A2 | 30-08-1999 |
| | | | | IL | 124880 | A | 12-01-2003 |
| | | | | JP | 3100401 | B2 | 16-10-2000 |
| | | | | JP | H11503653 | A | 30-03-1999 |
| | | | | KR | 19990076577 | A | 15-10-1999 |
| | | | | NO | 315456 | B1 | 08-09-2003 |
| | | | | PL | 327337 | A1 | 07-12-1998 |
| | | | | RU | 2189836 | C2 | 27-09-2002 |
| | | | | UA | 49865 | C2 | 15-10-2002 |
| | | | | US | 6544238 | B1 | 08-04-2003 |
| | | | | US | 2002138050 | A1 | 26-09-2002 |
| | | | | WO | 9723253 | A1 | 03-07-1997 |

EPO FORM P0459

For more details about this annex : see Official Journal of the European Patent Office, No. 12/82